# EUROPEAN PATENT APPLICATION

(11) **EP 4 040 270 A2**
(43) Date of publication of application: **10.08.2022**
(21) Application number: 22154433.1
(22) Date of filing: 01.02.2022
(51) Int. Cl.: G06F 3/01, G02B 27/01, G06F 3/03, G06F 3/0346, G06T 19/00

(54) **TRACKING SYSTEM**

(30) Priority: 03.02.2021 US 202163144995 P
(71) Applicant: HTC Corporation, Taoyuan City 330, (TW)
(72) Inventor: CHEN, Chih Chien, 330 Taoyuan City (TW)
(74) Representative: Klang, Alexander H.

(57) **Abstract**

The embodiments of the disclosure provide a tracking system. The tracking system includes a plurality of trackers, wherein each of the trackers includes a plurality of light emitting element groups, and each of the light emitting element groups includes a plurality of light emitting elements. Each of the trackers is configured to enable at least one of the light emitting element groups, light distribution patterns formed by the at least one of the enabled light emitting element groups on each of the trackers are different from each other, and hardware designs of the trackers are identical to each other.

## Description

### BACKGROUND

### 1. Field of the Invention

The present disclosure generally relates to a tracking mechanism, in particular, to a tracking system using light emitting elements.

### 2. Description of Related Art

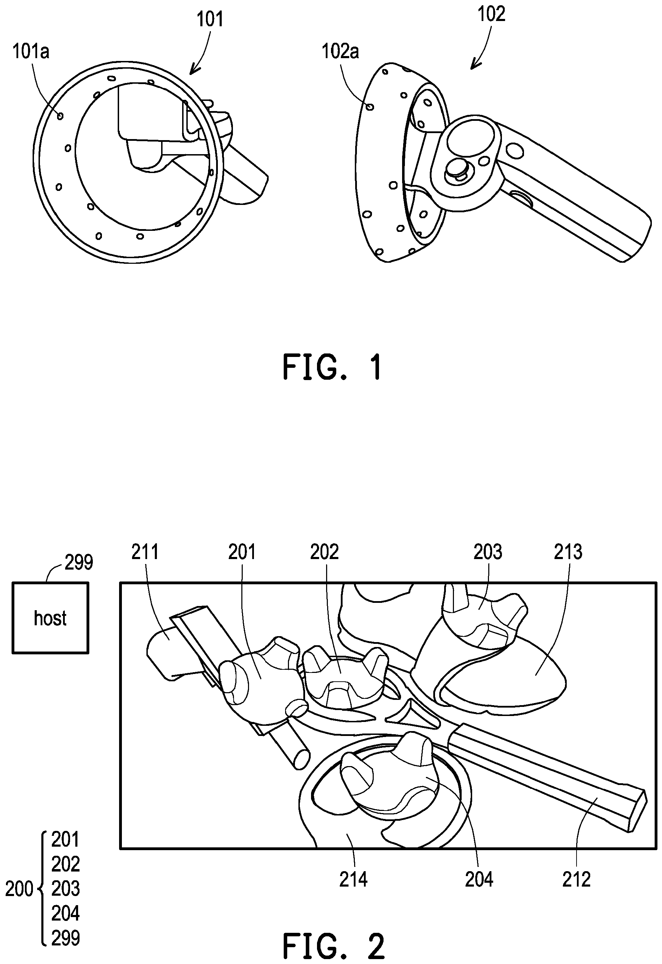

See FIG. 1, which shows a schematic diagram of to-be-tracked objects. In FIG. 1, the to-be-tracked objects 101 and 102 may be a pair of handheld controllers in a virtual reality (VR) system. In order to make the to-be-tracked objects 101 and 102 trackable, the to-be-tracked objects 101 and 102 can be respectively disposed with light emitting elements 101a and 101b (such as light emitting diodes (LED)) that emit lights (e.g., visible/invisible lights). In this case, the head-mounted display (HMD) of the VR system can determine the poses of the to-be-tracked objects 101 and 102 based on the light distributions of the light emitting elements 101a and 102a by using, for example, inside-out tracking mechanisms.

In the above way, in order to make the to-be-tracked objects 101 and 102 distinguishable to the HMD, the distribution of the light emitting elements 101a on the to-be-tracked object 101 and the distribution of the light emitting elements 101b on the to-be-tracked object 102 need to be different. That is, the hardware design of the to-be-tracked objects 101 and 102 cannot be the same, or the to-be-tracked objects 101 and 102 would be indistinguishable to the HMD

However, for trackers with identical hardware designs, the above ways cannot be applied.

### SUMMARY OF THE INVENTION

Accordingly, the disclosure is directed to a tracking system, which may be used to solve the above technical problems.

The embodiments of the disclosure provide a tracking system, including a plurality of trackers, wherein each of the trackers is trackable and includes a plurality of light emitting element groups, and each of the light emitting element groups includes a plurality of light emitting elements. Each of the trackers is configured to enable at least one of the light emitting element groups, light distribution patterns formed by the at least one of the enabled light emitting element groups on each of the trackers are different from each other, and hardware designs of the trackers are identical to each other.

The embodiments of the disclosure provide a tracking system, including at least one tracker, wherein each of the at least one tracker is trackable and includes a switch and a plurality of light emitting element groups, and each of the light emitting element groups includes a plurality of light emitting elements. The light emitting element groups on each of the at least one tracker include a fixed light emitting element group and a movable light emitting element group, a position of each light emitting element in the fixed light emitting element group is fixed, and a position of each light emitting element in the movable light emitting element group is switchable between a plurality of positions by using the switch.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the disclosure.
FIG. 1 shows a schematic diagram of to-be-tracked objects.
FIG. 2 shows a schematic diagram of a tracking system according to a first embodiment of the disclosure.
FIG. 3 shows a schematic diagram of a tracker according to the first embodiment of the disclosure.
FIG. 4 shows light distribution patterns on different trackers according to FIG. 3.
FIG. 5 shows a schematic diagram of light distributions on different trackers according to the first embodiment of the disclosure.
FIG. 6 shows a schematic diagram of switching positions of one of the light emitting element groups according to a second embodiment of the disclosure.
FIG. 7A is a schematic diagram of the light emitting elements on the tracker when the switch is in the first state according to the second embodiment of the disclosure.
FIG. 7B is a top view of FIG. 7B.
FIG. 7C is a cross-sectional view of FIG. 7B.
FIG. 8A is a schematic diagram of the light emitting elements on the tracker when the switch is in the second state according to FIG. 7A to FIG. 7C of the disclosure.
FIG. 8B is a top view of FIG. 8B.
FIG. 8C is a cross-sectional view of FIG. 8B.

### DESCRIPTION OF THE EMBODIMENTS

Reference will now be made in detail to the present preferred embodiments of the invention, examples of which are illustrated in the accompanying drawings. Wherever possible, the same reference numbers are used in the drawings and the description to refer to the same or like parts.

See FIG. 2, which shows a schematic diagram of a tracking system according to a first embodiment of the disclosure. In FIG. 2, the tracking system 200 includes, for example, trackers 201-204. In the embodiments of the disclosure, the trackers 201-204 can be disposed on and/or attached to different to-be-tracked objects 211-214, respectively. In addition, the hardware designs of the trackers 201-204 are identical to each other.

In one embodiment, the trackers 201-204 can be connected to a host 299 (e.g., the HMD in a VR system), and the host 299 can determine the poses of the to-be-tracked objects 211-214 based on the poses of the trackers 201-204.

In one embodiment, each of the trackers 201-204 is trackable and includes a plurality of light emitting element groups, and each of the light emitting element groups includes a plurality of light emitting elements (e.g., LEDs).

In one embodiment, each of the trackers 201-204 is configured to enable at least one of the light emitting element groups. In one embodiment, the host 299 connected to the trackers 201-204 can be used to control the trackers 201-204 to enable the corresponding light emitting element groups.

In the embodiments of the disclosure, the light emitting elements in an enabled light emitting element group can be controlled to emit lights, and the light emitting elements not in an enabled light emitting element group can be controlled to not emit lights.

In the embodiments of the disclosure, light distribution patterns formed by the enabled light emitting element groups on each of the trackers 201-204 are different from each other. In this case, the host 299 can distinguish the trackers 201-204 and accordingly determine the poses of the to-be-tracked objects 211-214.

In other embodiments, the tracking system 200 can include more or less trackers having identical hardware designs, but the disclosure is not limited thereto.

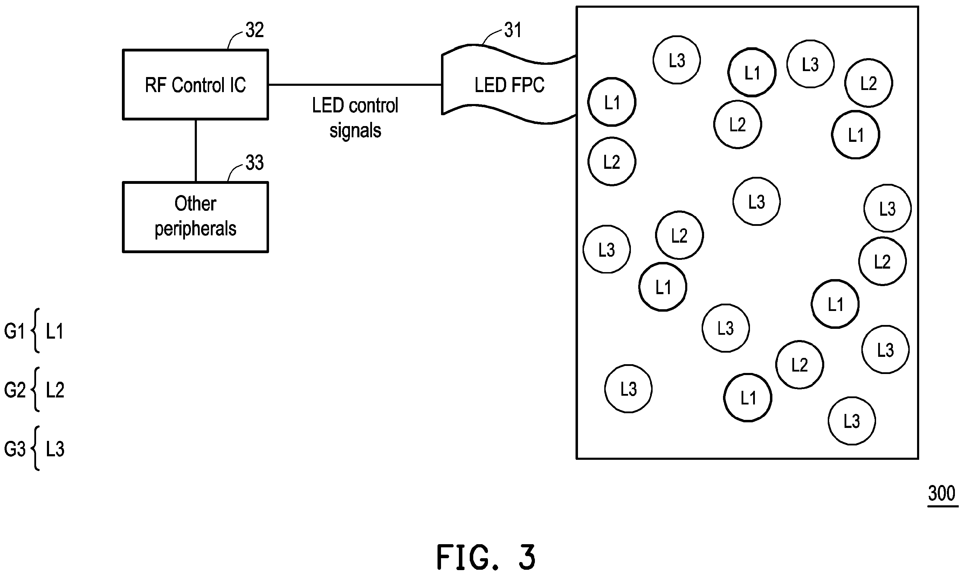

See FIG. 3, which shows a schematic diagram of a tracker according to the first embodiment of the disclosure. In FIG. 3, the tracker 300 can correspond to any of the trackers (e.g., the trackers 201-204) of the tracking system 200 in FIG. 2.

In the embodiments of the disclosure, the tracker 300 includes, for example, a LED flexible printed circuit board (FPC) 31, a radio frequency (RF) control integrated circuit (IC) 32, other peripherals 33, and light emitting element groups G1-G3, wherein the RF control IC 32 may provide LED control signals to the LED FPC 31 and other control signals to the other peripherals 33.

In FIG. 3, the light emitting element group G1 includes light emitting elements L1, the light emitting element group G2 includes light emitting elements L2, and the light emitting element group G3 includes light emitting elements L3, but the disclosure is not limited thereto.

In one embodiment, a first tracker (e.g., the tracker 201) of the tracking system 200 can be configured, by the host 299, to enable the light emitting element group G1, and the first tracker can be configured, by the host 299, to disable the light emitting element group G2. In addition, the second tracker (e.g., the tracker 202) is configured, by the host 299, to enable the light emitting element group G2, and the second tracker can be configured, by the host 299, to disable the light emitting element group G1. In some embodiments, the host 299 may send corresponding commands to the RF control IC 32 on each of the first tracker and the second tracker, such that the RF control IC 32 on each of the first tracker and the second tracker can provide corresponding LED control signals to the light emitting element groups thereof to enable/disable the required light emitting element groups, but the disclosure is not limited thereto.

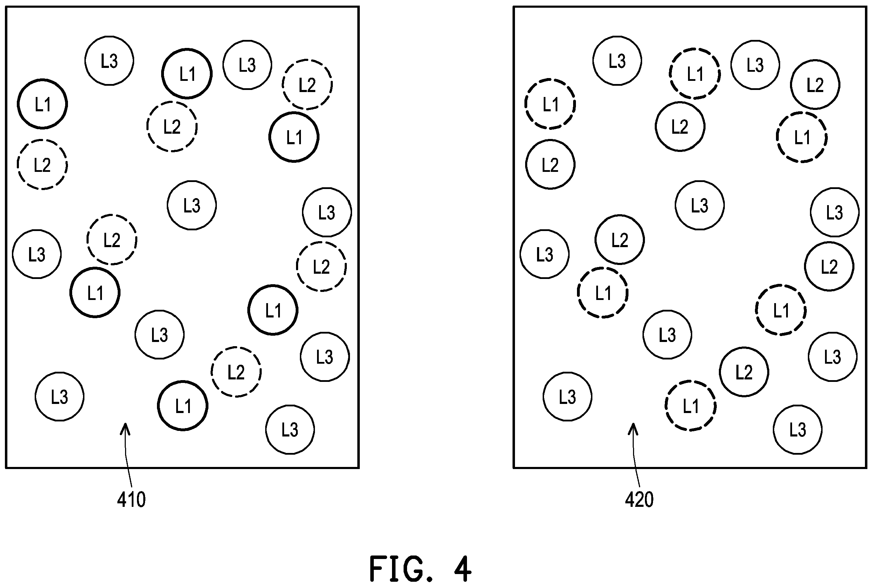

In one embodiment, the first tracker and the second tracker can be further configured, by the host 299, to enable the light emitting element group G3, which would be discussed with FIG. 4.

See FIG. 4, which shows light distribution patterns on different trackers according to FIG. 3. In FIG. 4, the disabled light emitting element groups are shown in dotted lines for visual aid. As shown in FIG. 4, a light distribution pattern 410 can be formed by the enabled light emitting element groups G1 and G3 on the first tracker, and a light distribution pattern 420 can be formed by the enabled light emitting element groups G2 and G3 on the second tracker.

As can be observed in FIG. 4, the light distribution pattern 410 is different from the light distribution patter 420. In this case, the host 299 can distinguish the first tracker and the second tracker, and accordingly determine the poses of the corresponding to-be-tracked objects (e.g., the to-be-tracked objects 211 and 212).

In some embodiments, the light emitting element group G3 on each of the first tracker and the second tracker can be disabled if the numbers of the light emitting elements in the light emitting element groups G1 and G2 are enough for the host 299 to track. In this case, the light distribution pattern formed by the enabled light emitting element group G1 on the first tracker is still different from the light distribution pattern formed by the enabled light emitting element group G2 on the second tracker, and the host 299 can still distinguish the first tracker and the second tracker, and accordingly determine the poses of the corresponding to-be-tracked objects (e.g., the to-be-tracked objects 211 and 212). Further, a third tracker (e.g., the tracker 203) in the tracking system 200 can be configured, by the host 299, to enable the light emitting element group G3 (and disable the light emitting element groups G1 and G2), such that the host 299 can distinguish the first tracker, the second tracker, and the third tracker, and accordingly determine the poses of the corresponding to-be-tracked objects (e.g., the to-be-tracked objects 211-213), but the disclosure is not limited thereto.

In one embodiment, the host 299 can include a camera (e.g., a front camera of the HMD) used to capture images of the trackers of the tracking system 200. In this case, the host 299 can determine the poses of the trackers based on the light distribution patterns captured in the images by using, for example, the inside-out tracking mechanisms, which can be referred to relevant prior arts. Afterwards, the host 299 can accordingly determine the poses of the corresponding to-be-tracked objects, but the disclosure is not limited thereto.

In some embodiments, the trackers in the tracking system 200 can be implemented in the forms other than those shown in FIG. 2. For example, the form/type of the trackers can be implemented as handheld controllers (e.g., the handheld controller in FIG. 1), bracelet, or the like. In these cases, the trackers can be hold and used by hands of the user, such that the pose/gesture of the hands of the user can be determined by the host 299.

In some embodiments, each tracker of the tracking system 200 can be disposed with inertial measurement units (IMUs) to provide more fine grain orientation to the host 299.

In some embodiments, each tracker of the tracking system 200 can include communication circuits (e.g., RF interfaces) used to communicate with the host 299 via protocols such as Bluetooth or the like, but the disclosure is not limited thereto.

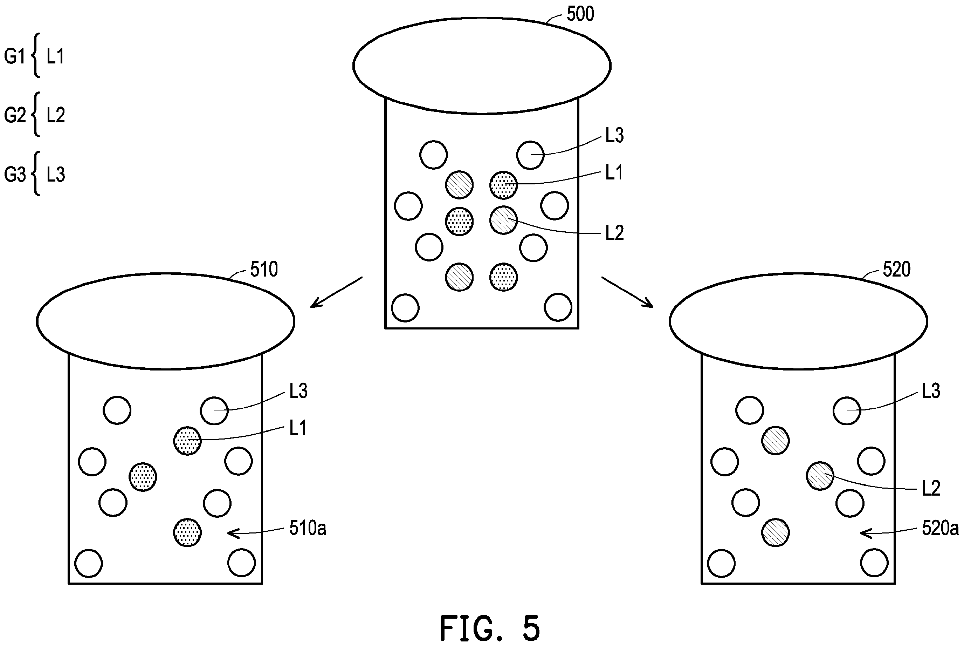

In some embodiments, the position of the light emitting elements in the light emitting element group G1 and G2 can be symmetrical as shown in FIG. 5.

See FIG. 5, which shows a schematic diagram of light distributions on different trackers according to the first embodiment of the disclosure. In FIG. 5, the distributions of the light emitting element groups G1-G3 on each of trackers 500 (which may be implemented as a handheld controller) of the tracking system 200 are exemplarily shown, wherein the light emitting elements L1 in the light emitting element group G1 are shown as circles with dots, the light emitting elements L2 in the light emitting element group G2 are shown as circles with oblique lines, and the light emitting elements L3 in the light emitting element group G3 are shown as empty circles. As can be observed in FIG. 5, the distribution of the light emitting elements L1 in the light emitting element group G1 is symmetrical to the distribution of the light emitting elements L2 in the light emitting element group G2.

In the scenario of FIG. 5, the host 299 may disable the light emitting element group G2 and enable the light emitting element groups G1 and G3 on one tracker 510 of the trackers 500, such that the light distribution pattern the tracker 510 can be presented as the light distribution pattern 510a in FIG. 5, wherein the disabled light emitting element group G2 is not shown in the light distribution pattern 510a. In addition, the host 299 may disable the light emitting element groups G1 and enable the light emitting element groups G2 and G3 on another tracker 520 of the trackers 500, such that the light distribution pattern on the tracker 520 can be presented as the light distribution pattern 520a in FIG. 5, wherein the disabled light emitting element group G1 is not shown in the light distribution pattern 510b. In this case, the light distribution pattern 510a can be symmetrical to the light distribution pattern 520a, and the trackers 510 and 520 can be held by the hands of the user for pose/gesture detection, but the disclosure is not limited thereto.

In some embodiment, trackers with the same hardware design can be configured to have different light distribution patterns via the following solutions.

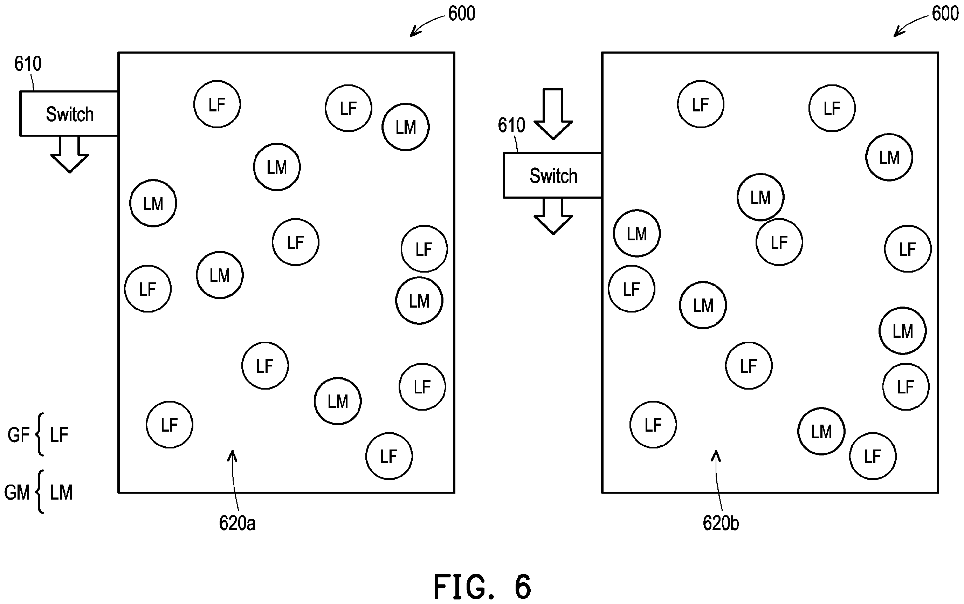

See FIG. 6, which shows a schematic diagram of switching positions of one of the light emitting element groups according to a second embodiment of the disclosure. In the second embodiment, the trackers in a tracking system have the same hardware design. In FIG. 6, each of the trackers in the tracking system of the second embodiment of the disclosure has a switch 610 and a plurality of light emitting element groups. In the second embodiment, the light emitting element groups on each tracker include a fixed light emitting element group GF and a movable light emitting element group GM, wherein a position of each light emitting element LF in the fixed light emitting element group GF is fixed, and a position of each light emitting element LM in the movable light emitting element group GM is switchable between a plurality of positions by using the switch 610.

In the second embodiment, the switch 610 can be implemented as a hardware switch that can be changed to be in a first state and a second state. In the second embodiment, the switch 610 can be in a on state (e.g., the first state) or an off state (e.g., the second state), but the disclosure is not limited thereto.

In the second embodiment, the position of each light emitting element LM in the movable light emitting element group GM is switchable between a first position and a second position via the switch 610. In the second embodiment, the switch 610 in the first state controls the position of each light emitting element LM in the movable light emitting element group GM to be the first position, and the switch 610 in the second state controls the position of each light emitting element LM in the movable light emitting element group GM to be the second position.

In the second embodiment, the switch 610 on the left side of FIG. 6 can be regarded as in the first state. Accordingly, the positions of each light emitting element LM in the movable light emitting element group GM are in the corresponding first position. On the other hand, the switch 610 on the right side of FIG. 6 can be regarded as in the second state. Accordingly, the positions of each light emitting element LM in the movable light emitting element group GM are in the corresponding second position.

In the second embodiment, each light emitting element LF in the fixed light emitting element group GF and each light emitting element LM in the movable light emitting element group GM are enabled to emit lights.

In one embodiment, the switch 610 in a first tracker of the tracking system can be configured to be in the first state, which makes a first specific light distribution pattern 620a formed by the fixed light emitting element group GF and the movable light emitting element group GM on the first tracker can be presented as shown on the left side of FIG. 6. In addition, the switch 610 in a second tracker of the tracking system can be configured to be in the second state, which makes a second specific light distribution pattern 620b formed by the fixed light emitting element group GF and the movable light emitting element group GM on the second tracker can be presented as shown on the right side of FIG. 6.

In this case, the first specific light distribution pattern and the second specific light distribution pattern 620b provided by the first tracker and the second tracker are different, such that the host (e.g., the HMD) connected with the first tracker and the second tracker is able to distinguish the first tracker and the second tracker in the images captured by the camera of the host.

In the second embodiment, the host can determine the poses of the first tracker and the second tracker based on the first specific light distribution pattern 620a and the second light distribution pattern 620b captured in the images of the camera. Therefore, the host can determine the poses of the first tracker and the second tracker, and accordingly determine the poses of the to-be-tracked objects on which the first tracker and the second tracker are respectively attached.

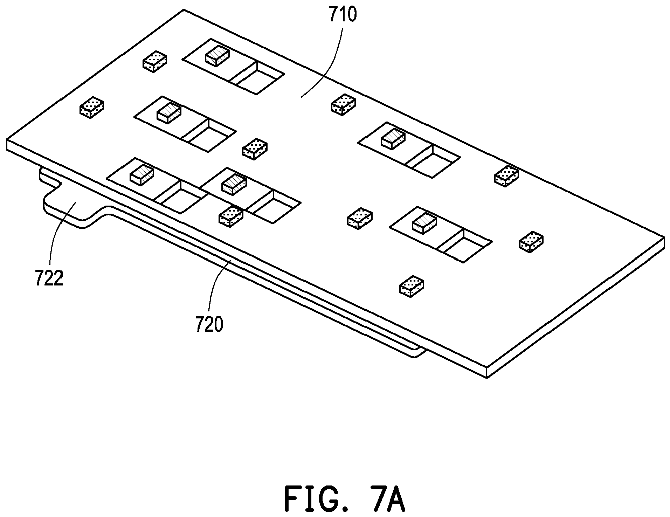

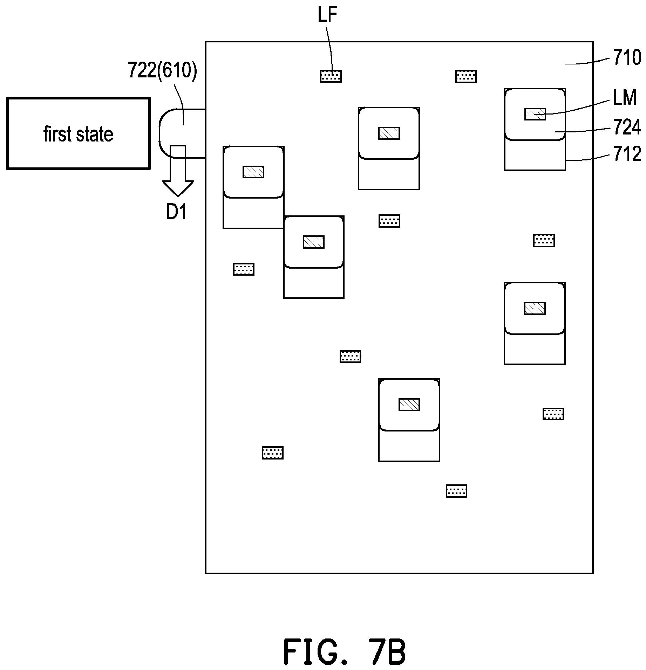

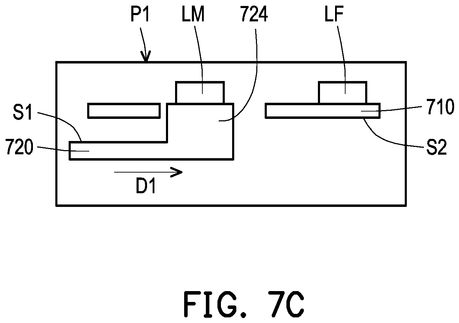

See FIG. 7A to FIG. 7C, wherein FIG. 7A is a schematic diagram of the light emitting elements on the tracker when the switch is in the first state according to the second embodiment of the disclosure, FIG. 7B is a top view of FIG. 7B, and FIG. 7C is a cross-sectional view of FIG. 7B.

In FIG. 7A to FIG. 7C, the tracker includes a first circuit board 710 and a second circuit board 720, wherein the light emitting elements LF (shown as boxes with dots) in the fixed light emitting element group GF can be disposed on the first circuit board 710.

The second circuit board 720 has a first protruding portion 722 disposed at a side of the second circuit board 720, which can be used as the switch 610 mentioned in the above. In addition, the second circuit board 720 has multiple second protruding portions 724 disposed on a first surface S1 of the second circuit board 720, and the light emitting elements LM (shown as boxes with oblique lines) in the movable light emitting element group GM are disposed on the second protruding portions 724.

In one embodiment, the first circuit board 710 has multiple openings 712, and each of the openings 712 can be used to accommodate one of the second protruding portions 724. In particular, the second circuit board 720 can be disposed under the first circuit board 710, such that the first surface S1 of the second circuit board 720 faces a second surface S2 of the first circuit board 710. In addition, the positions of the second protruding portions 724 can correspond to the positions of the openings 712, and the sizes of the second protruding portions 724 can be smaller than the corresponding openings 712.

In one embodiment, the second protruding portions 724 can be moved in the corresponding openings 712 in response to the movement of the switch 610 (i.e., the first protruding portion 722).

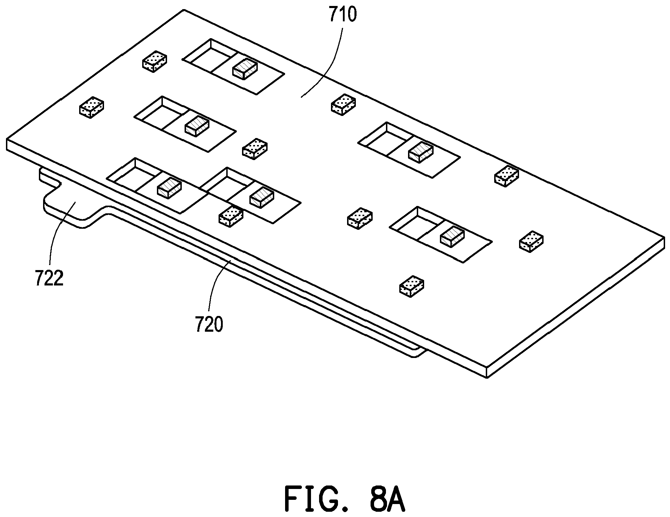

In FIG 7A to FIG. 7C, the switch 610 can be assumed to be in the first state, which represents that each of the light emitting elements LM is currently in the first position P1. In this case, the switch 610 can be moved along a direction D1 to become the second state. In FIG. 7B, when the switch 610 is moved along the direction D1 (i.e., a downward direction), the second protruding portions 724 would be moved downward accordingly. In this case, each of the light emitting elements LM are moved from the first position P1 to be the second position P2, which are shown in FIG. 8A to FIG. 8C.

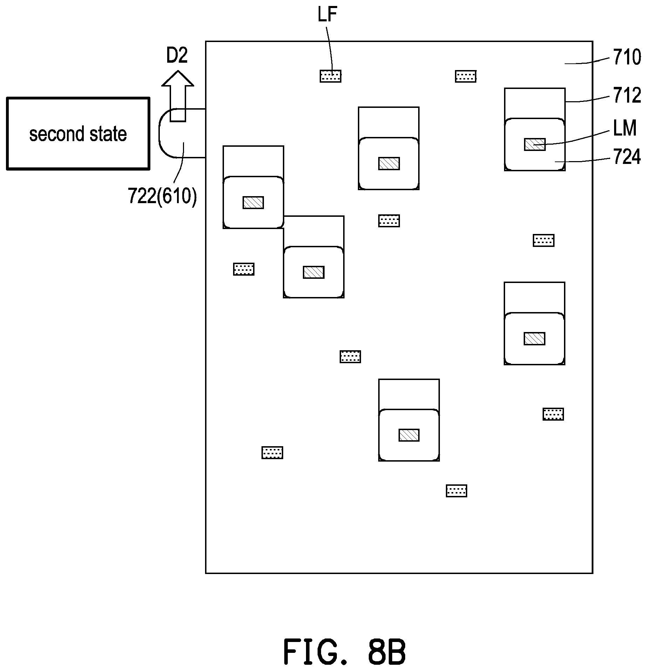

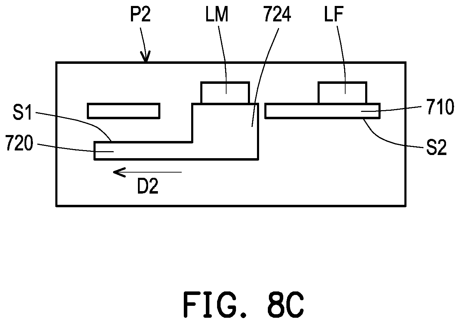

See FIG. 8A to FIG. 8C, wherein FIG. 8A is a schematic diagram of the light emitting elements on the tracker when the switch is in the second state according to FIG. 7A to FIG. 7C of the disclosure, FIG. 8B is a top view of FIG. 8B, and FIG. 8C is a cross-sectional view of FIG. 8B.

As can be observed in FIG. 7B and FIG. 8B, the light distribution pattern formed under the circumstance where the switch 610 is in the first state is different from the light distribution pattern formed under the circumstance where the switch 610 is in the second state.

In FIG. 8A to FIG. 8C, the switch 610 can be assumed to be in the second state, which represents that each of the light emitting elements LM is currently in the second position P2. In this case, the switch 610 can be moved along a direction D2 to become the first state. In FIG. 8B, when the switch 610 is moved along the direction D2 (i.e., an upward direction), the second protruding portions 724 would be moved upward accordingly. In this case, each of the light emitting elements LM are moved from the second position P2 to be the first position P1, i.e., the scenario shown in FIG. 7A to FIG. 7C.

In one embodiment, the switch 610 of the first tracker of the tracking system of the second embodiment can be configured to be in the first state as shown in FIG. 7A to FIG. 7C. In addition, the switch 610 of the second tracker of the tracking system of the second embodiment can be configured to be in the second state as shown in FIG. 8A to FIG. 8C.

In this case, the light distribution patterns in FIG. 7B and FIG. 8B provided by the first tracker and the second tracker are different, such that the host (e.g., the HMD) connected with the first tracker and the second tracker is able to distinguish the first tracker and the second tracker in the images captured by the camera of the host.

In the second embodiment, the host can determine the poses of the first tracker and the second tracker based on the light distribution patterns in FIG. 7B and FIG. 8B captured in the images of the camera. Therefore, the host can determine the poses of the first tracker and the second tracker, and accordingly determine the poses of the to-be-tracked objects on which the first tracker and the second tracker are respectively attached.

In summary, the embodiments of the disclosure provide solutions to make the trackers with identical hardware designs have different light distribution patterns, such that the host can distinguish the trackers and determine the poses of the trackers and/or the corresponding to-be-tracked objects. For example, different parts of the light emitting elements on different trackers can be configured to be different, such that the trackers with identical hardware designs have different light distribution patterns.

In addition, the positions of a part of the light emitting elements can be moved by using a switch, such that the trackers whose switches are in different states can have different light distribution patterns.

It will be apparent to those skilled in the art that various modifications and variations can be made to the structure of the present invention without departing from the scope or spirit of the disclosure. In view of the foregoing, it is intended that the present disclosure cover modifications and variations of this invention provided they fall within the scope of the following claims and their equivalents.

## Claims

1. A tracking system, comprising:
a plurality of trackers, wherein each of the trackers is trackable and comprises:
a plurality of light emitting element groups, and each of the light emitting element groups comprises a plurality of light emitting elements;
wherein each of the trackers is configured to enable at least one of the light emitting element groups, light distribution patterns formed by the at least one of the enabled light emitting element groups on each of the trackers are different from each other, and hardware designs of the trackers are identical to each other.

2. The tracking system according to claim 1, wherein the trackers comprise:
a first tracker, wherein the light emitting element groups of the first tracker comprise a first light emitting element group and a second light emitting element group;
a second tracker, wherein the light emitting element groups of the second tracker comprise the first light emitting element group and the second light emitting element group;
wherein the first tracker is configured to enable the first light emitting element group, the second tracker is configured to enable the second light emitting element group, and a first light distribution pattern formed by the first light emitting element group on the first tracker is different from a second light distribution pattern formed by the second light emitting element group on the second tracker.

3. The tracking system according to claim 2, wherein the light emitting element groups of the first tracker and the second tracker further comprise a third light emitting element group, and the first tracker and the second tracker are further configured to enable the third light emitting element group, wherein a third light distribution pattern formed by the first light emitting element group and the third light emitting element group on the first tracker is different from a fourth light distribution pattern formed by the second light emitting element group and the third light emitting element group on the second tracker.

4. The tracking system according to claim 2, wherein the second light emitting element group on the first tracker is disabled, and the first light emitting element group on the second tracker is disabled.

5. The tracking system according to claim 1, further comprising a host configured to determine the at least one of the enabled light emitting element groups on each of the trackers.

6. The tracking system according to claim 5, wherein the host is a head-mounted display.

7. The tracking system according to claim 5, wherein the host is configured to perform:
determining poses of the trackers based on the light distribution patterns formed by the at least one of the enabled light emitting element groups on each of the trackers.

8. The tracking system according to claim 5, wherein the host comprises at least one camera used to capture images of the trackers, and the host is configured to perform:
determining the poses of the trackers based on the light distribution patterns captured in the images.

9. A tracking system, comprising:
at least one tracker, wherein each of the at least one tracker is trackable and comprises:
a switch;
a plurality of light emitting element groups, and each of the light emitting element groups comprises a plurality of light emitting elements;
wherein the light emitting element groups on each of the at least one tracker comprise a fixed light emitting element group and a movable light emitting element group, a position of each light emitting element in the fixed light emitting element group is fixed, and a position of each light emitting element in the movable light emitting element group is switchable between a plurality of positions by using the switch.

10. The tracking system according to claim 9, wherein the plurality of positions comprises a first position and a second position, the switch has a first state and a second state, the switch in the first state controls the position of each light emitting element in the movable light emitting element group to be the first position, and the switch in the second state controls the position of each light emitting element in the movable light emitting element group to be the second position.

11. The tracking system according to claim 10, wherein the at least one tracker comprises a first tracker and a second tracker, and hardware designs of the at least one tracker are identical to each other.

12. The tracking system according to claim 11, wherein the switch on the first tracker is in the first state, the switch on the second tracker is in the second state, a first specific light distribution pattern formed by the light emitting element groups on the first tracker is different from a second specific light distribution pattern formed by the light emitting element groups on the second tracker.

13. The tracking system according to claim 12, further comprising a host configured to perform:
determining poses of the first tracker and the second tracker based on the first specific light distribution pattern and the second light distribution pattern, respectively.

14. The tracking system according to claim 13, wherein the host is a head-mounted display.

15. The tracking system according to claim 13, wherein the host comprises at least one camera used to capture images of the first tracker and the second tracker, and the host is configured to perform:
determining the poses of the first tracker and the second tracker based on the first specific light distribution pattern and the second light distribution pattern captured in the images.
